(19) Europäisches Patentamt / European Patent Office / Office européen des brevets

(11) **EP 3 293 509 B1**

(12) **EUROPEAN PATENT SPECIFICATION**

(45) Date of publication and mention
of the grant of the patent:
**20.01.2021 Bulletin 2021/03**

(21) Application number: **16789359.3**

(22) Date of filing: **05.05.2016**

(51) Int Cl.:
*G01N 17/02* *(2006.01)*       *G01N 33/38* *(2006.01)*

(86) International application number:
**PCT/ES2016/070346**

(87) International publication number:
**WO 2016/177929 (10.11.2016 Gazette 2016/45)**

(54) **METHOD FOR DETERMINING THE CORROSION IN A REINFORCED CONCRETE STRUCTURE**

VERFAHREN ZUR BESTIMMUNG DER KORROSION BEI EINER BEWEHRTEN BETONSTRUKTUR

PROCÉDÉ POUR DÉTERMINER LA CORROSION DANS UNE STRUCTURE DE BÉTON ARMÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.05.2015 ES 201530614**

(43) Date of publication of application:
**14.03.2018 Bulletin 2018/11**

(73) Proprietor: **Universitat Politècnica de València**
**46022 Valencia (ES)**

(72) Inventors:
- **ALCAÑIZ FILLOL, Miguel**
**46022 Valencia (ES)**
- **BATALLER PRATS, Roman**
**46022 Valencia (ES)**
- **GANDIA ROMERO, Jose Manuel**
**46022 Valencia (ES)**

- **RAMON ZAMORA, Jose Enrique**
**46022 Valencia (ES)**
- **SOTO CAMINO, Juan**
**46022 Valencia (ES)**
- **VALCUENDE PAYA, Manuel Octavio**
**46022 Valencia (ES)**

(74) Representative: **Maslanka Kubik, Dorota Irena**
**C/Vara de Rey 5 TER, 3, Oficina 5**
**26003 Logroño (La Rioja) (ES)**

(56) References cited:
**JP-A- 2007 240 481     JP-A- 2011 089 871**
**JP-A- 2014 062 782     US-A1- 2012 043 981**

- **BASTIDAS D M ET AL.: 'quantitative study of concrete-embeddedsteel corrosion using potentiostatic pulses.' CORROSION vol. 63, no. 12, 01 December 2007, pages 1094 - 1100, XP001510575**

**Description**

TECHNICAL FIELD

The present invention relates to the field of construction, and more specifically to the determination of the state of reinforced concrete structures, specifically the determination of the state of corrosion thereof.

PRIOR ART

The most common way of determining the corrosion of frames in a reinforced concrete structure today is based on destructive techniques, which involve uncovering the frame every certain distance (depending on the number of points of the structure to be tested) to take the electrochemical measurement of the intensity of corrosion. Those actions must then be reverted by means of repair mortars.

The most widely used electrochemical methods include the linear polarization sweeping method and galvanostatic methods. Both methods require the use of an external guard ring which is used to locally confine the applied signal the reference electrode carries therein, an auxiliary electrode surrounding the reference electrode.

As regards calculation of the intensity of corrosion ($I_{CORR}$) by means of the linear polarization sweeping technique, there is a need to estimate parameter B of the Stern-Geary equation (described hereinbelow), where significant errors may therefore be made when predicting structure durability.

The use of sensors incorporated inside a reinforced concrete structure for monitoring the corrosion of said structure is also known in the prior art. For example, patent document CN 103852488 discloses a device embedded in the concrete. The device comprises a sensor and an external circuit. The sensor assures that the resonant frequency changes so as to evaluate the degree of corrosion of the frame by means of on/off situations of different steel wires based on an oscillating circuit. The module allows wireless transmission of the collected data. However, the design of this sensor is relatively complex, and failures may therefore surface over time. Furthermore, although it allows monitoring the state of corrosion in the location in which it is placed, it does not allow assuring that the frame of the reinforced concrete exhibits the same state of corrosion in any other location.

Patent document JP 2007240481 discloses a sensor for determining the corrosion in a reinforced concrete structure, comprising a sensor element, a temperature sensor and conductors electrically connecting the sensor element, the temperature sensor as well as the frame to a connection box. Different ways for securing and electrically connecting the sensor to the frame are known in the prior art. For example, in the patent document JP 2011089871, the sensor is mounted on a fixture that fixes a first reinforcing bar and a second reinforcing bar. Patent document JP 2014062782 discloses the use of a clamp for electrically connecting the frame.

Bastidas et al. ("A quantitative study of concrete-embedded steel corrosion using potentiostatic pulses". Corrosion (December 2007), National Assoc. of Corrosion Engineers International. Vol. 63, no. 12, pages 1094 - 1100) disclose a quantitative study of concrete-embedded steel corrosion using potentiostatic pulses. The described method is independent of the surface area of the steel under study, providing that the double-layer capacitance value is known. It also requires the use of small counter electrodes. However, this method is not as reliable as in the case in which the surface area that is being studied is accurately known.

Therefore, there is still a need in the prior art for an alternative method for determining the corrosion of a frame in a reinforced concrete structure, which allows obtaining reliable results in a quick and simple manner.

DISCLOSURE OF THE INVENTION

The present invention provides a method as defined by claim 1.

BRIEF DESCRIPTION OF THE DRAWINGS

The present invention will be better understood in reference to the following drawings which illustrate preferred embodiments of the invention that are provided by way of example and must not be interpreted as limiting the invention in anyway.

Figure 1 shows a side view of a sensor used in the preferred embodiment of the present invention, installed in a region of the frame of a reinforced concrete structure.

Figure 2 is a top view of the sensor shown in Figure 1.

Figure 3 is a perspective view of a reinforced concrete structure with a sensor coupled to a region of the frame thereof.

Figure 4 is a schematic depiction of measuring equipment for use in the method according to the specific embodiment of the present invention.

Figure 5 is a graph of a train of potentiostatic pulses applied according to the method of the preferred embodiment of the present invention.

Figure 6 is a depiction of the equivalent circuit used for fitting the curves of intensity over time in a method according to the preferred embodiment of the present invention.

Figure 7 is a graph depicting the calculation of the intensity of corrosion based on partial $R_p$ values obtained by means of a pulse train according to a preferred embodiment of the present invention.

Figure 8 is a schematic depiction in a side view of an arrangement for measuring $E_{CORR}$ (V) with the external measuring equipment arranged on a reinforced concrete structure.

Figures 9, 10 and 11 are graphs showing a comparison of $R_p$, $i_{corr}$ and $E_{corr}$ values, respectively, obtained by means of the method of the present invention, with values obtained by means of a linear sweep voltammetry technique by means of introducing sensor elements in solution.

Figures 12, 13 and 14 are graphs showing a comparison of $R_p$, $i_{corr}$ and $E_{corr}$ values, respectively, obtained by means of the method of the present invention, with values obtained by means of a linear sweep voltammetry technique, in mortar and steel-reinforced concrete test pieces.

Figure 15 is a graph depicting mass loss over time predicted by means of the method of the present invention, in comparison with experimental measurements obtained by means of gravimetry.

Figures 16, 17 and 18 are graphs showing a comparison of $R_p$, $R_s$ and $i_{corr}$ values, respectively, obtained by means of the method of the present invention using a 2-electrode and 3-electrode configuration.

## DETAILED DISCLOSURE OF THE INVENTION

First, the present invention uses both a sensor and a network formed by a plurality of said sensors, for taking non-destructive measurements of the state of corrosion of a steel frame in a reinforced concrete structure. With the information obtained from the sensor network placed in the structure of a building, the general state thereof and the possible problems that may arise locally or in general can therefore be known, such that maintenance can be optimized. The obtained information will allow predicting well in advance any intervention deemed necessary, where repair and maintenance costs are significantly reduced.

The sensors can be applied, for example, in new construction works (incorporating them at the time of pouring the concrete in those regions of the structure that are the most exposed to corrosion caused by the penetration of depassivating ions (chlorides), $CO_2$ and moisture), in intervention works (allowing control and subsequent non-destructive follow-up on the effectiveness of the repair) or in laboratories (for research work with test pieces to attain further understanding of the corrosion processes of concrete-embedded frames).

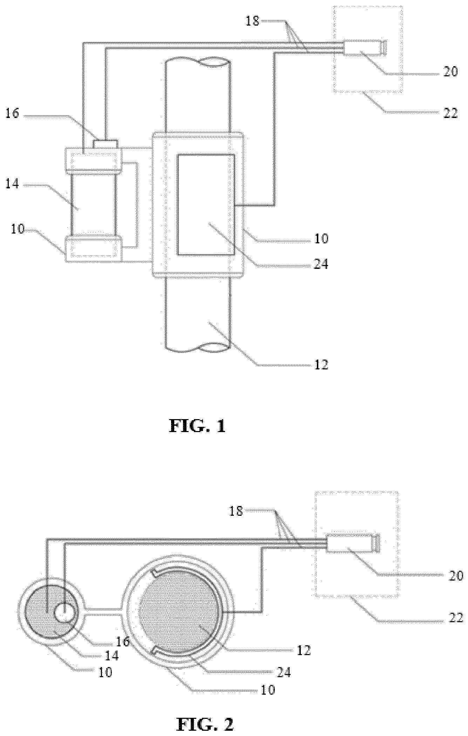

The sensor depicted in Figures 1 and 2, comprises:

- a support (10) for coupling the sensor to a bar of the frame (12) of the reinforced concrete structure. The support (10) is preferably manufactured with (rigid or flexible) ceramic or polymeric materials and is designed with a suitable geometry which places the sensor in the most suitable position with respect to the metal frame (12) to be controlled.
- a sensor element (14) secured to the frame (12) by means of the support (10).

This sensor element (14) acts as a working electrode when taking voltammetric or potentiometric measurements according to the method of the present invention, as will be described hereinbelow. It is formed by a small fragment of the same material as the frame installed at the site, with a known surface. According to the preferred embodiment, the surface of the sensor element (14) measures 5 to 30 cm$^2$.

- a temperature sensor (16) which can be, for example, a thermocouple, NTC, PTC, RTP or a semiconductor temperature sensor, adhered to the support (10) on the surface thereof with resins (for example, LM50B), or embedded in the support (10) itself.
- conduction and connection elements (18) which allow electrically insulating and connecting the frame (12), the sensor element (14) and the temperature sensor (16) as required and transporting the signal to an external connector acting as an electrical switch which allows connecting the sensor element (14) to the metal frame (12) to be controlled and disconnecting it from same.

- a metal connecting clamp (24) for connecting the frame (12) with the conduction and connection elements. The clamp (24) can be fitted by means of a mechanical snap-fit or screwed-on closure with an inert material made of externally insulated stainless steel, nylon nuts and bolts or polymers of similar characteristics or nylon zip ties, for example. The clamp contains therein two semicircular metal connectors attached to an insulated wire which, with the pressure exerted by the clamp (24), assure the electrical contact between the frame (12) and a connection box (22). The support (10) described above protects the clamp (24) from moisture.

- the external connector attached to the sensor element (14) and to the frame (12) by means of the conduction and connection elements (18). It is formed by a connector (20) inside a small connection box (22), embedded in the surface of the concrete structure. Information can be obtained by means of a jack for connecting to measuring equipment which will obtain the reading of the local temperature, the corrosion potential and the intensity of corrosion from the sensor. It can also have a mechanism for the connection of a reference electrode.

Sensor geometry may obviously vary in different embodiments of the present invention depending on the point of application and on the diameter of the frames to assure a good contact therewith.

A sensor network can be formed just by installing a plurality of sensors described hereinabove in a respective plurality of locations in the frame of a reinforced concrete structure. As many parts of the structure as desired can thus be monitored to thereby know the general state of the structure.

One or more sensor elements can be integrated in the same region by means of a common connection, adopting various configurations (matrix, aligned, etc.) which allow recording the changes in local properties existing inside the reinforced concrete in a simultaneous manner and at several points.

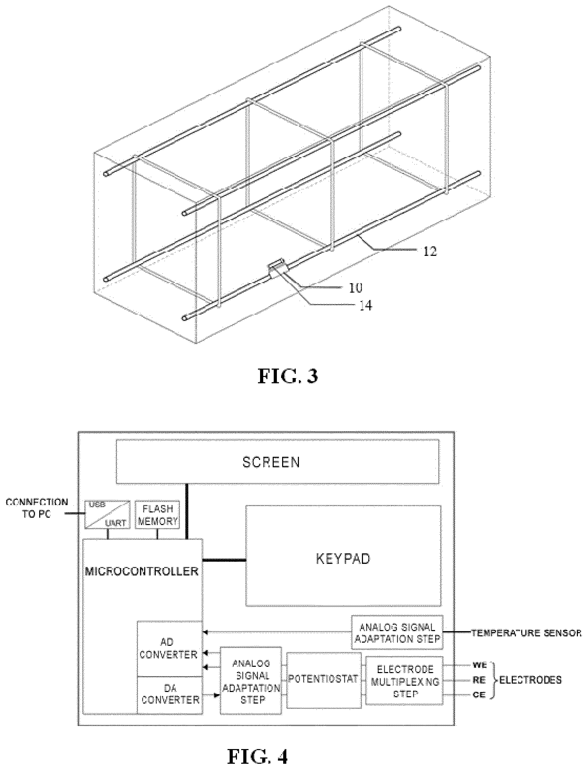

Figure 3 shows an example of installing a sensor in a frame (12) inside a reinforced concrete structure. For the sake of simplicity, this figure only shows one sensor, but the person skilled in the art will understand that a plurality of sensors can be installed in the same frame (12) to form the sensor network described above.

The method of operating the sensor, according to the preferred embodiment of the present invention will be described below. The external connector acts as an electrical switch which allows connecting the sensor element (14) to the metal frame (12) to be controlled and disconnecting it from same. The connector can therefore be found in a normal state (with the sensor element (14) connected to the metal frame (12)), and a sensing state (with the sensor element (14) disconnected from the metal frame (12)).

The normal state involves direct and local connection of the sensor element (14) in a region of the metal frame (12) inside the reinforced concrete structure. In that situation, it is affected by the physicochemical conditions of its environment, acquiring an electric potential which is the local electric potential of that region of the frame (12) and must be conferred by the actual physicochemical parameters of the concrete matrix in which it is embedded. Those determining factors are, for example, the local concentration of oxygen entering by diffusion through the pore or capillary system of the concrete, the pH value depending on the manufacturing parameters and on the degree of material carbonation, on the moisture of the region, on the presence of aggressive anions such as chlorides or sulfates, on the temperature, etc. In the normal state, the sensor element (14) will have corrosion potential and speed similar to those of the frame (12) in the region with which it is connected.

The sensing state involves the sensor element (14) being disconnected from the metal frame (12) by means of the introduction of an external jack in the external connector on the surface. An independent electrical contact for each element is thereby achieved and information about the local state of the frame (12) with which it was connected can be obtained.

In this situation, information is obtained from the sensor element (14) in a non-destructive manner through a jack connector (i.e., it is not necessary to break the concrete of the region to reach the frame (12) and establish an electrical connection). Use of an external auxiliary electrode is not required when working in this manner, since the actual metal frame (12) of the concrete, through the second wire of the jack, acts as a non-polarizable auxiliary electrode. The guard ring that is commonly used for locally confining the electric signal in a region of the frame (12) during determination of the corrosion rate is not required either, since the surface of the sensor element (14) is accurately known.

The measurement can be taken in ordinary working conditions by working with 2 electrodes (the sensor element (14) and the frame (12)) without external reference because due to the high surface-area ratio of the embedded frame (12) with respect to the sensor element (14), the latter can act as a non-polarizable auxiliary electrode. So the actual reinforcement frame (12) of the reinforced concrete structure can be considered an auxiliary electrode and a reference electrode. By working with 2 electrodes, the overpotential that is applied coincides with the amplitude of the designed pulse train. According to another preferred embodiment of the present invention, it is also possible to work with 3 electrodes (connecting an external reference electrode in the external connector) for determining the corrosion rate, in which case an end result which will be the same but with the values normalized by said external reference electrode will be obtained.

As described above, the main advantage is that when working with the 2-electrode technique, the sensor allows monitoring hard-to-access regions of the structure, such as submerged or buried elements, in which an auxiliary electrode or an external reference electrode cannot be used. An accurate and reliable measurement of the corrosion rate that is representative of the state of the critical regions of the structure can thus be obtained.

In order to obtain information generated from the sensor (local temperature, corrosion potential (OCP) and intensity of corrosion), external measuring equipment which can work with conductimetric, potentiometric and voltammetric techniques, which can work with two and three electrodes and with a total of 5 different information management channels with a multiplexing system is required.

The method according to the preferred embodiment of the present invention therefore comprises the following steps:

a) connecting external measuring equipment, as defined above, to the external connector of the sensor. Connection between the external measuring equipment and each of the sensor element, the temperature sensor and the frame is thereby established. In other words, the sensor transitions to the sensing state described hereinabove.

b) determining the temperature of the sensor element based on the measurement obtained from the temperature sensor, for example the resistance measurement of the temperature sensor in the case of a PT100-type temperature sensor.

c) determining the electrical resistance conferred by the concrete. It is an estimative measurement obtained by applying a low-amplitude square wave (preferably between 20 and 200 mV) working at a specific frequency (preferably chosen from 1 and 10 KHz) between the working electrode of the sensor and the metal frame of the concrete. The data obtained can be used for designing the duration of the voltammetric pulses that are used later on, as will be described in detail hereinbelow.

d) determining the corrosion potential (OCP, "open circuit potential") value by means of a potentiometric measurement of the difference in electric potential between a working electrode of the sensor and a reference electrode (ECS, Ag/AgCl, Cu/SO$_4$Cu or any other electrode to be used). Specifically, and as will be described in detail hereinbelow, the potentiometric measurement of the difference in electric potential between the electrodes is taken at regular time intervals (for example, intervals of 10 to 20 s), where the values may or may not be displayed on a screen as a function of time. When the variation between the measured value and the earlier value is less than a given threshold for 5 consecutive measurements, the average of said 5 measurements is calculated and that average is considered as the corrosion potential value.

According to an embodiment of the present invention, this step of the method can also be performed with a 2-electrode technique (i.e., the reference electrode is the actual frame of the reinforced concrete structure, instead of an external reference electrode). In this case, the value which is obtained is therefore the difference in relative potential between the metal frame and the sensor, providing an idea of the anodic or cathodic behavior of the region of the sensor with respect to the rest of the metal frame.

e) applying pulse trains for calculating the polarization resistance and intensity of corrosion. In this step, the equipment starts to work voltammetrically with the 2-electrode or 3-electrode technique, as described hereinabove. In the first case (2 electrodes), a multiplexor of the measuring equipment causes the sensor element to act as a working electrode and the metal frame to act as an auxiliary/reference electrode. In the case of working with the 3-electrode technique, an external reference electrode which is kept in contact with the concrete surface closest to the sensor, for example through a sponge wetted with water, is furthermore connected. A series of potentiostatic pulse trains which allow determining the following is then applied:

D1: The polarization resistance (Rp) of the system. Based on the previously calculated OCP, a series of low-amplitude pulses which allow obtaining the Rp value with high reliability is applied. The degree of polarization of the system is at least ten times less than that produced by applying a potential sweep technique commonly used today for the determination thereof.

D2: The intensity of corrosion. By means of a pulse train that is specially designed to that end (described hereinbelow), the intensity of corrosion is determined based on the protocol proposed by Tafel. The coincidence between the values obtained using conventional Tafel sweeping and the pulse train method is highly accurate (greater than 99%). However, the observed polarization of the sensor is 90% less when the pulse method according to the present invention is used in comparison with the potential sweep method. This is in accordance with the total load that comes into play in each of the techniques.

As described above, the first step of the method of the present invention requires connecting measuring equipment. Figure 4 shows a schematic depiction of external measuring equipment which can be used in the embodiment of the method of the present invention. Said measuring equipment includes, for example, the following elements:

▪ A display screen, for example of an LCD-type display screen, on which the progression of the measurements as well as the result thereof is displayed.

▪ A keypad whereby the user can configure, start or cancel the measurements. According to an additional embodiment of the present invention, the keypad and the display screen are combined in a single element, such as a touch screen, for example.

▪ A flash memory in which the measurement data is stored to be subsequently downloaded into a computer.

▪ A UART to USB converter allowing communication between a microcontroller of the measuring equipment and the computer.

▪ An analog signal adaptation step for the temperature sensor which converts the electric signal generated by the temperature sensor into a voltage the interval of which corresponds with the input interval of an analog-digital converter (ADC) of the microcontroller.

▪ An electrode multiplexing step which allows connecting and disconnecting the electrodes with respect to the rest of the circuitry depending on the type of measurement that is taken. This step allows the potentiostat to take voltammetry measurements and potentiometry measurements.

▪ A potentiostat which allows controlling the voltage applied between the reference electrode (RE) and the working electrode (WE) in the voltammetric measurements. Furthermore, the potentiostat provides the ADC (AD converter) of the microcontroller (through a step of adapting the corresponding analog signal) with the voltage between the WE and the RE, as well as the current circulating through the WE.

▪ An analog signal adaptation circuit step for adjusting the current and voltage signals coming from the potentiostat to the input interval of the ADC and adapting the output of the digital-analog converter (DAC) of the microcontroller to the voltage levels that must be applied to the electrodes.

▪ A microcontroller in charge of reading the data entered using the keypad, showing the corresponding information on the display screen, coordinating the execution of the measurement sequence by selecting the corresponding electrodes at all times, generating the analog signal that must be applied to the electrodes, digitizing the analog signals from the temperature sensor and those corresponding to the voltage and current in the electrodes, processing the measurement data by making the corresponding calculations and storing the results in the flash memory to then send them to a computer.

The measuring equipment is powered with a battery and incorporates the additional circuitry required for generating the voltages for powering all the circuits.

For example, according to an embodiment of the present invention, measuring equipment as described hereinabove can be used for determining the polarization resistance and intensity of corrosion on each floor of a building having several floors. The method then comprises the step of sending the polarization resistance value and the intensity of corrosion value that are determined by the external measuring equipment to a central control unit of the building by means of wireless connection.

A more detailed description of the steps run by the CPU of the measuring equipment based on the instructions included in the computer program after connecting the measuring equipment to the external connector of the sensor according to the present invention is provided below.

As mentioned above, first the temperature of the environment is measured. To that end, the microcontroller performs an analog-digital conversion of the voltage generated by the temperature sensor, converts the obtained digital value into a temperature value, preferably shows the result on the display screen and stores it in a non-volatile memory.

The resistance conferred by the concrete between the sensor and the frame ($R_s$) is then determined. To that end, the microcontroller generates by means of the digital-analog converter (DAC) a train of, for example, 10 square wave pulses with a peak-to-peak value between 20 mV and 200 mV, an offset of 0 V and a frequency between 1 and 10 KHz, preferably 10 kHz. Said voltage is applied to the working electrode (WE) through the potentiostat. Simultaneously, the microcontroller, through the analog-digital converter (ADC), measures the current (1 sample every 10 $\mu$s) in the working electrode and computes an average for each pulse. It then obtains the average current for 10 pulses ($I_{AVERAGE}$) and calculates with that value the $R_S$ value according to Equation 1 shown below:

$$R_S = \frac{V_P}{I_{AVERAGE}}$$

(1)

where $V_P$ is the amplitude of the pulses (between 20 mV and 200 mV).

The $R_S$ value is stored in a non-volatile memory and is also preferably shown on the screen.

This data is used together with the capacitance of the electrical double layer for determining the pulse width value (s), where the pulse train to be applied in a subsequent step can be configured beforehand.

The corrosion potential (OCP) value is then determined. To that end, the microcontroller configures the potentiostat in the potentiometry mode, and by means of the ADC, reads the potential value between the working electrode "sensor element" and the reference electrode (Ag/AgCl, Cu/CuSO$_4$, etc.) or the frame of the concrete structure. For each reading, the microcontroller compares the read value with the earlier value. When the difference between the read value and the earlier value for 5 consecutive measurements is less than a specific threshold (established in the configuration), the microcontroller stops the measuring process and stores the average value of the last 5 measurements as OCP. The OCP value is stored in a non-volatile memory and is also preferably shown on the screen.

If work is to be performed with 2 electrodes, since a standard external reference electrode is not used, the values obtained are close to 0 volts given that the reference is the reinforcement frame itself, the local OCP of which and the OCP of the sensor element itself will usually be very similar. This does not affect the corrosion measurement, since this value is only taken as a reference for applying the corresponding overpotential in the pulse train.

A pulse train is then applied. When the potentiostat is configured in the working mode with 3 electrodes, the microprocessor converts the sensor element into a working electrode, the frame into an auxiliary electrode and also uses the chosen reference electrode. It then uses the previously measured OCP and a sequence is started in which the current going through the system is measured until it is below a specific threshold (established in the configuration of the equipment). Once this point is reached, the microcontroller starts a new measurement sequence in which it applies, in a preferred embodiment, the following pulse trains on the working electrode:
OCP/OCP + 10 mV/OCP/OCP - 10 mV/OCP, followed by: OCP/OCP + 70 mV/OCP/OCP - 70 mV/OCP, ending with: OCP/OCP + 140 mV/OCP/OCP - 140 mV/OCP.

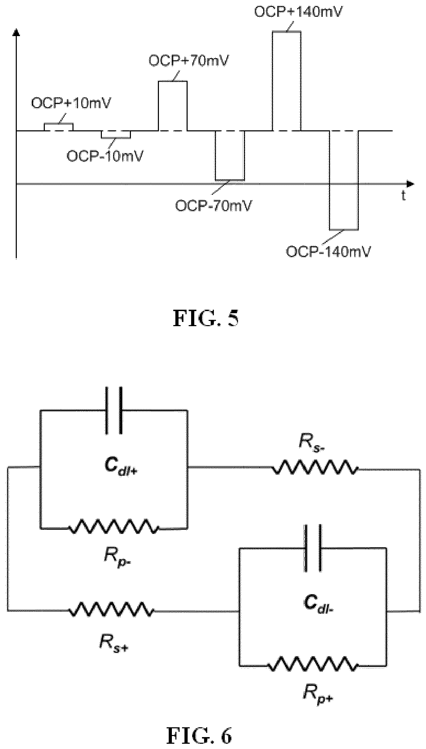

These three pulse sequences can be combined in a single pulse train as shown in the attached Figure 5.

If the potentiostat is configured in the working mode with 2 electrodes, the sensor element acts as a working electrode and the frame acts as an auxiliary electrode and as a reference electrode. In these conditions, the previously measured OCP with respect to the frame is applied and a sequence is started in which the current going through the system is measured until it is below a specific threshold (established in the configuration of the equipment).

After applying each pulse, the microcontroller measures the current until the variation of the value thereof in 5 consecutive samples is below a specific threshold. The current value is considered stable at that moment. The duration of each of the applied pulses will depend on the time it takes for the current to stabilize. The values of the progression of the current over time as well as the stabilization value (calculated by computing the average of the last 5 measurements) are stored in a non-volatile memory.

The preceding application of the pulse train mainly serves for calculating the polarization resistance and intensity of corrosion. To that end, the equivalent circuit that best describes the corrosion process occurring in the structure for the system with 2 electrodes (see the attached Figure 6) is considered, based on which equations of the intensity circulating therethrough are developed. The total current is the sum of the current derived from load transfer (faradaic current $I_f$) and the current due to load reorganization in the electrical double layer (non-faradaic current $I_{dl}$). Therefore, the intensity-time curve obtained by applying the potentiostatic pulse is the resulting sum of the intensity-time curves of the faradaic and non-faradaic current.

By solving the circuit of Figure 6, the following two equations, which allow evaluating the faradaic and non-faradaic contribution of anodic and cathodic currents going through the sensor element and the auxiliary electrode as a function of time, are obtained.

$$I_{WE} = \frac{V_{AC}}{R_{pWE} + R_{sWE}}\left(1 - e^{-(1 + R_{sWE}/R_{pWE})t/R_{sWE}C_{WE}}\right) + \frac{V_{AC}}{R_{SWE}}e^{-(1 + R_{sWE}/R_{pWE})t/R_{sWE}C_{WE}}$$

$$(2)$$

$$I_{EA} = \frac{V_{AC}}{R_{pEA} + R_{sEA}}\left(1 - e^{-(1 + R_{sEA}/R_{pEA})t/R_{sEA}C_{EA}}\right) + \frac{V_{AC}}{R_{SEA}}e^{-(1 + R_{sEA}/R_{pEA})t/R_{sEA}C_{EA}}$$

$$(3)$$

The total intensity is equal to the sum of the four terms appearing in the preceding equations. The subscript WE appearing in the first equation refers to the intensity going through the working electrode, whereas the subscript EA refers to the auxiliary electrode. By fitting the experimental data of the intensity as a function of time for the +/-10, +/-70 and +/-140 mV pulses to the proposed equations, the numerical value of each of the elements that form the equivalent circuit for the electrode and are used for calculating the following parameters is obtained:

Density of corrosion using the polarization resistance (Rp) of the system, *icorr (Rp):* based on the $R_p$ and $R_s$ values obtained by fitting the +10 mV and -10 mV pulses to the equivalent circuit, $R_p'$ is calculated for the anodic branch (Equation 4) and cathodic branch (Equation 5), which have the same value (Equation 6). Total $R_p'$ is calculated with

Equation 7.

$$R'_{pWE} = R_{pWE} + R_{sWE}$$

(4)

$$R'_{pEA} = R_{pEA} + R_{sEA}$$

(5)

$$R'_{pWE} = R'_{pEA}$$

(6)

$$R'_{pTOTAL} = \frac{R'_{pWE}}{2} = \frac{R'_{pEA}}{2}$$

(7)

Based on this total $R_p$' value, the stabilization intensity at infinite time corresponding to the positive and negative pulse is calculated according to Equation 8:

$$i = \eta E/R'_{pTOTAL}$$

(8)

where $\eta E$ is the overpotential applied with respect to the OCP, in this case +0.01 and - 0.01 V, respectively. The intensity values of the positive pulse (+10 mV) and negative pulse (-10 mV) with respect to the corresponding overpotential are then represented and the inverse of the slope of the line joining them is the final Rp' to be considered. This parameter can also be calculated in a simplified manner (without fitting data to the equivalent circuit) from the Rp' of the anodic and cathodic branch (Equations 4 and 5) using the $R_p$ calculated according to Equation 9 by using the average stabilization intensity (SI) of +10 and -10 mV pulses, substituting them into Equation 9:

$$R_P = \frac{0.01\,V}{I_{AVERAGE\_S}}$$

(9)

The corrosion current value is obtained by means of the Stern-Geary formula shown in the following Equation 10:

$$I_{CORROSION}\left(R_p\right) = \frac{B}{R_p}$$

(10)

where B can take a value between 13 mV and 52 mV (in this case a value of 26 mV is chosen). The density of corrosion $i_{CORR}$ (A/cm$^2$) is obtained by dividing the $I_{CORR}$ (A) value by the surface of the sensor element.

For the measurement system using 2 electrodes, the overpotential that is applied coincides with the amplitude of the designed pulse train; when working with 3 electrodes, such amplitude must be substituted in the equations by the overpotential that is applied between the working electrode and the auxiliary electrode.

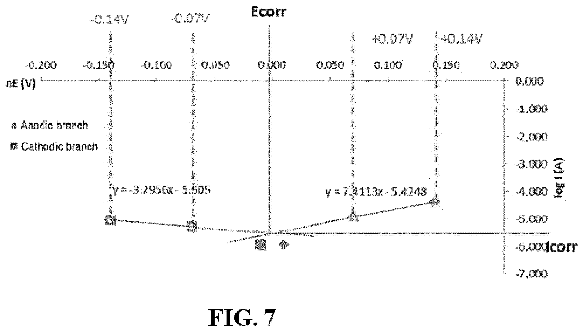

Density of corrosion of the structure based on simplifying the Tafel extrapolation method, $i_{CORR}$ (Tafel pulses): using the Rp values obtained by fitting the +70 mV, -70 mV, +140 mV and -140 mV pulses to the equivalent circuit, the Rp' corresponding to each of them is calculated. The corresponding current intensity is then calculated according to the preceding Equation 8 and these values are represented as intensity log vs. corresponding overpotential (see Figure 7). The line/curve anodic Tafel equation (the slope of which is $\beta_{ANODIC}$) is obtained based on the currents for +70 mV and +140 mV pulses, and the line/curve cathodic Tafel equation (the slope of which is $\beta_{CATHODIC}$) is obtained based on the currents for -70 mV and -140 mV pulses. The x-axis of the intersecting point between both lines corresponds with $E_{CORR}$ (V), whereas the y-axis corresponds with the corrosion current $I_{CORR}$ (A). The density of corrosion $i_{CORR}$ (A/cm$^2$) is obtained by dividing this parameter by the known surface of the sensor element. Additionally, Equation (11) links $\beta_{ANODIC}$

and $\beta_{CATHODIC}$ with the Stern-Geary constant B. Figure 7 and Table I below show an example of this calculation.

TABLE I

| | | PULSE | $P_p$ (Ω) | x - E (V) | y - i (A) | y - Log(i) |
|---|---|---|---|---|---|---|
| Anodic line | A | P+70 | 1 810.472 | 0.070 | 3.866E-05 | -4.413 |
| | B | P+140 | 2 010.664 | 0.140 | 6.963E-05 | -4.157 |
| Cathodic line | A' | P-70 | 1 572.341 | -0.070 | -4.452E-05 | -4.351 |
| | B' | P-140 | 1 470.390 | -0.140 | -9.521 E-05 | -4.021 |

All this data is stored in a non-volatile memory and is preferably shown on the screen.

$$B = \frac{\beta_A \cdot \beta_C}{2.303 \cdot (\beta_A + \beta_C)} \qquad (11)$$

For the measurement system using 2 electrodes, the overpotential that is applied coincides with the amplitude of the designed pulse train; when working with 3 electrodes, such amplitude must be substituted in the equations by the overpotential that is applied between the working electrode and the auxiliary electrode.

Corrected local corrosion potential of the structure, corrected $E_{CORR}$ (V): the local corrosion potential $E_{CORR}$ (V) referring to the OCP of the metal frame is obtained in the calculation of the preceding step. To allow comparing this data with corrosion potentials from other studies, generally obtained with 3-electrode systems, the value is corrected so that it refers to a standard reference electrode (ECS, Ag/AgCl, Cu/SO$_4$Cu, etc.). One of these reference electrodes (as needed) is used to replace the preceding reference electrode (metal frame), which is now the working electrode.

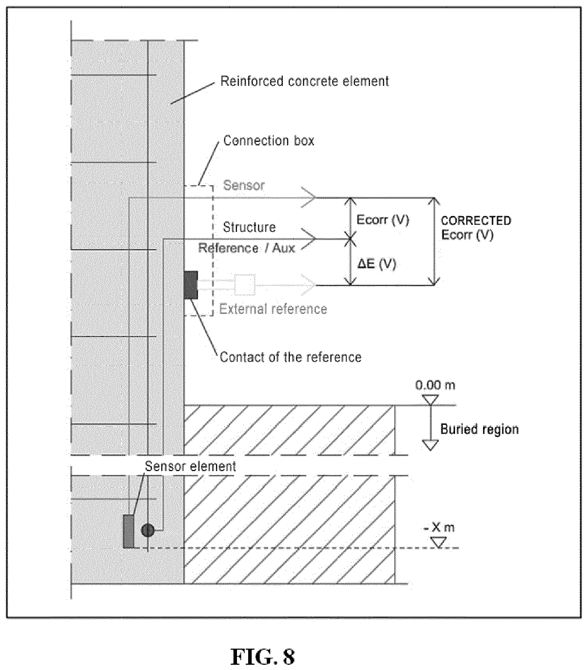

The chosen reference is placed in an accessible region close to the external connector, on the surface of the concrete as shown in Figure 8. With this new arrangement, the measuring equipment repeats the step of determining the corrosion potential value described above, thereby obtaining the difference in potential between the frame and the new reference. This value, along with the $E_{CORR}$ (V) value obtained in the preceding step, results in the corrected $E_{CORR}$ (V).

Finally, the data can be downloaded to a PC through the USB connection incorporated in the measuring equipment. A PC application allows downloading, displaying and storing the collected data. For each test the test identifier and the associated measurement data, such as that listed below, are downloaded:

- Test identifier
- Temperature
- Resistance of the concrete (Rs)
- Corrosion potential (OCP)
- Values of the samples of the progression of the current signal in the working electrode over time when voltammetric pulses are applied thereto
- $R_P$ value
- $R_P$' value
- Corrosion current value by means of the $R_P$ method with B = 26 mV: $I_{CORR}(R_P)$. Corrosion current limit values by means of the $R_P$ method for B = 13 mV and B = 52 mV.
- Value of the slopes of the $\beta_{ANODIC}$ and $\beta_{CATHODIC}$ Tafel line/curve. B value calculated from $\beta_{ANODIC}$ and $\beta_{CATHODIC}$. B (Tafel pulses).
- Value of the x-intercept of the Tafel lines/curves which is equivalent to the corrosion potential: $E_{CORR}$ (Tafel pulses).
- Value of the y-intercept of the Tafel lines/curves which is equivalent to the corrosion current: $I_{CORR}$ (Tafel pulses).

Finally, studies have been conducted for validating the measurements of the density of corrosion obtained by means of the method disclosed by the present invention with respect to those obtained by means other conventional techniques such as cyclic voltammetry and linear sweep voltammetry (Rp).

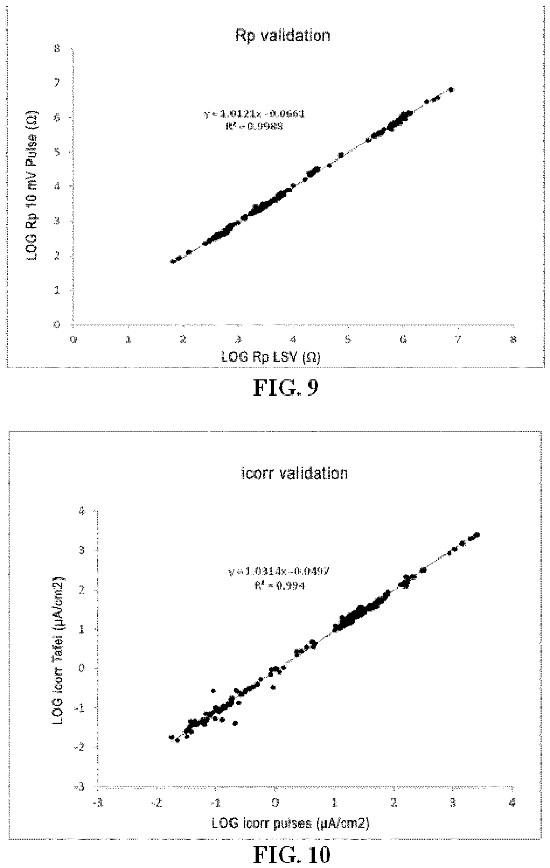

Studies were conducted with S275JR and B500SD steel and zinc in solution, in which the electrodes manufactured according to the present invention were submerged in the presence of chloride anions (0.5 M), sulfate anions (0.1 M), nitrate anions (0.1 M) and carbonate anions (0.1 M) at different pH values (7, 9, 11 and 13), large intervals of density of corrosion and corrosion potential thereby being obtained. Figure 9 shows the log values of the polarization resistance (Ω) calculated by means of the method disclosed in the present invention $\pm 10$ mV with respect to the values obtained by means of linear sweep voltammetry (LSV) in the interval from -10 mV to +10 mV at a sweeping speed of 0.16 mV/second.

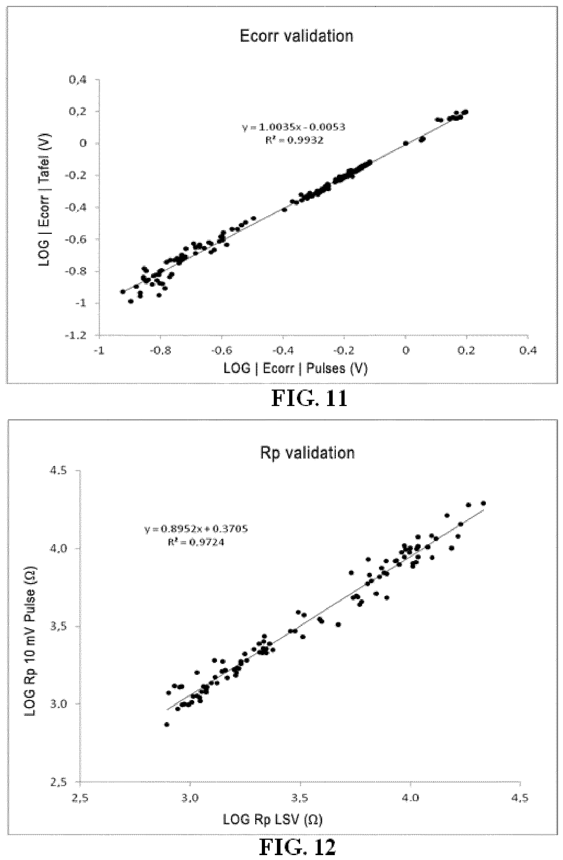

Figure 10 shows the log values of the density of corrosion, $i_{CORR}$, calculated using the Tafel linear sweep voltammetry method in comparison with the method disclosed in the present invention. Figure 11 compares the resulting $E_{CORR}$ value obtained by means of both methods.

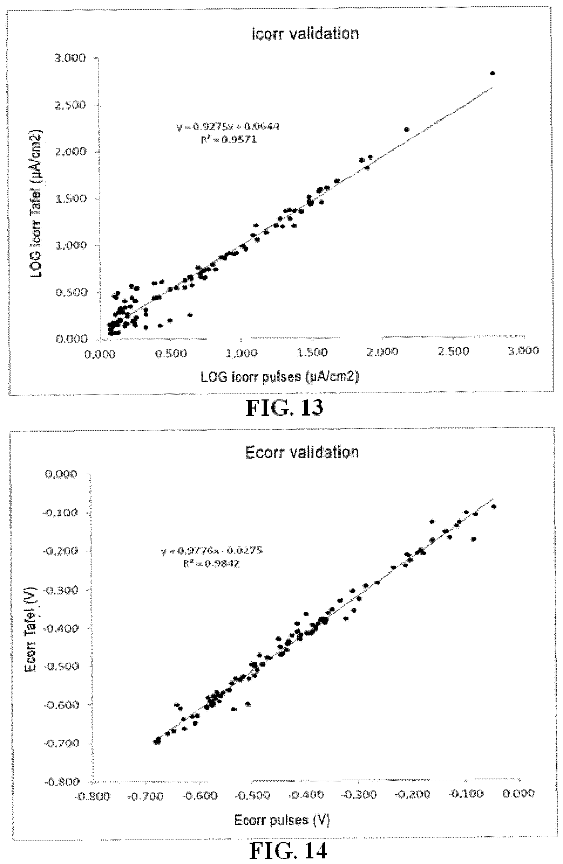

The electrochemical techniques proposed in mortar and B500SD steel-reinforced concrete test pieces were applied in the same manner. The samples were also attacked with chloride (0.5 M), sulfate (0.1 M), nitrate (0.1 M) and carbonate (0.1 M), large intervals of density of corrosion and corrosion potential thereby being obtained. Figures 12, 13 and 14 compare the obtained $R_P$, $i_{CORR}$ and $E_{CORR}$ values, respectively, for the studied techniques.

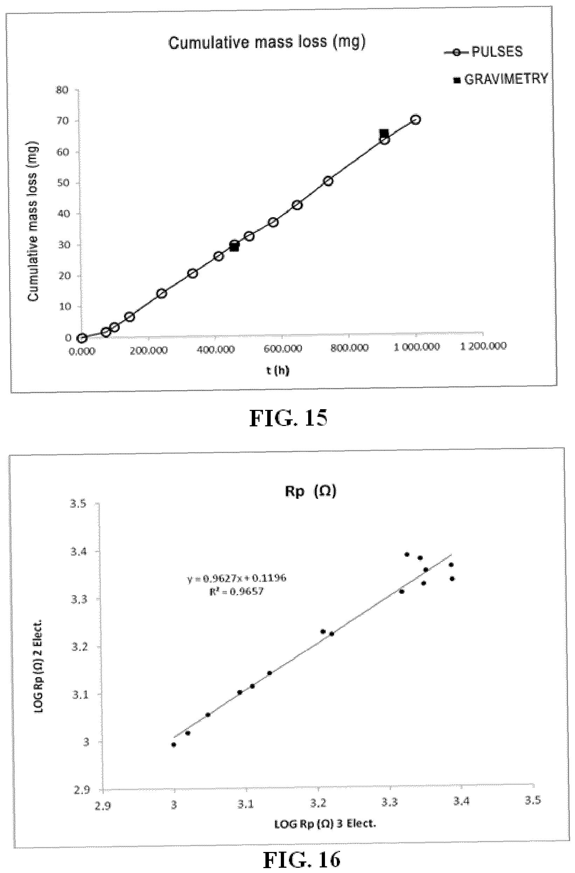

Furthermore, the potentiostatic pulse technique is validated against gravimetry in three S275JR steel bars, where an actual mass loss due to corrosion equal to that obtained by means of the voltammetric pulse technique of the method according to the present invention is observed (Figure 15).

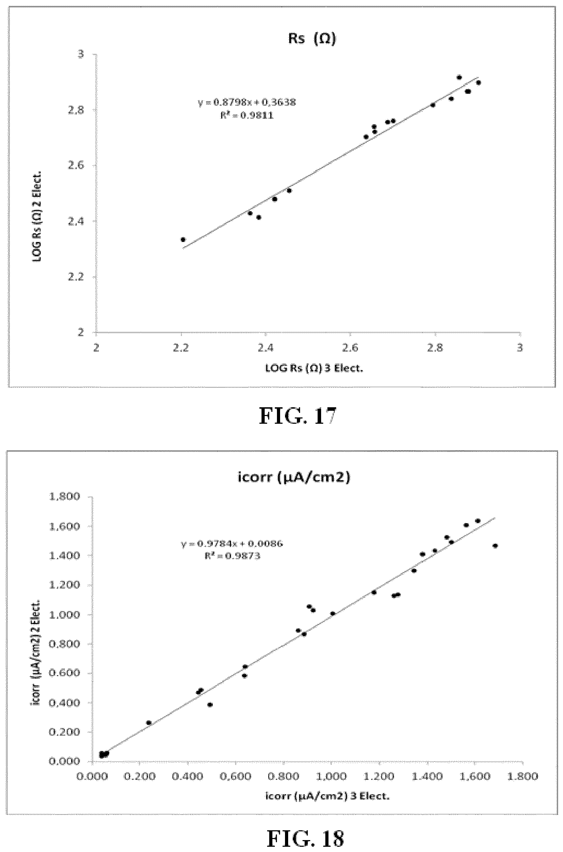

Finally, the reproducibility of the values obtained with the measurement system with 2 electrodes is also validated against the configuration with 3 electrodes in test pieces with embedded corrosion sensor prototypes. Figures 16, 17 and 18 show, respectively, comparisons of the measurements of the values of the polarization resistance (Rp), resistance of the concrete (Rs), density of corrosion ($i_{CORR}$) using 2 and 3 electrodes. These graphs show that, in both configurations, the method according to the present invention provides comparable results.

Although the present invention has been described in reference to preferred embodiments thereof, modifications and variations based on the teachings provided herein will occur to those skilled in the art without departing from the scope of the present invention described in the following claims.

For example, although an embodiment of the method using pulse trains with potential values of +/-10 mV, +/-70 mV and +/-140 mV has been described, one skilled in the art will understand that in alternative embodiments of the present invention different potential values for the pulse trains can also be used without departing from the scope of the present invention as a result.

**Claims**

1. A method of determining the corrosion in a reinforced concrete structure by means of using at least one sensor incorporated in the reinforced concrete structure, said sensor comprising:

   - a support (10) coupling the sensor to a bar of the frame (12) of the concrete structure;
   - a sensor element (14) secured to the frame (12) by means of the support (10);
   - a temperature sensor (16);
   - conduction and connection elements (18) which allow electrically insulating or connecting the frame (12), the sensor element (14) and the temperature sensor (16) by means of an external connector that is arranged to act as an electrical switch;
   - a metal connecting clamp (24) connecting the frame (12) with the conduction and connection elements (18);
   - the external connector attached to the sensor element (14) and to the frame (12) by means of the conduction and connection elements (18), and formed by a connector (20) inside a connection box (22) embedded in the surface of the concrete structure;

   **characterized in that** the method comprises the steps of:

   a) connecting external measuring equipment to the external connector of the sensor, establishing the connection between the external measuring equipment and each of the sensor element (14), the temperature sensor (16) and the frame (12);
   b) determining the temperature of the sensor element (14) based on the measurement obtained from the temperature sensor (16);
   c) determining the electrical resistance conferred by the concrete;
   d) determining the corrosion potential value by means of a potentiometric measurement of the difference in electric potential between a working electrode of the sensor and a reference electrode;
   e) applying pulse trains to the sensor, for calculating the polarization resistance and intensity of corrosion;
   f) obtaining information from the sensor; and
   g) calculating the polarization resistance and intensity of corrosion.

2. The method according to claim 1, **characterized in that** the sensor element (14) is manufactured from the same material as the frame (12) of the concrete structure and has a surface measuring 5 to 30 cm$^2$.

3. The method according to any of the preceding claims, **characterized in that** the external connector is furthermore arranged for the connection of the reference electrode.

4. The method according to any of the preceding claims, **characterized in that** the temperature sensor (16) is selected from the group consisting of a thermocouple, NTC, PTC, RTP or a semiconductor temperature sensor.

5. The method according to any of the preceding claims, **characterized in that** it is carried out by means of using a sensor network formed by a plurality of sensors arranged in a respective plurality of locations in the frame (12) of the reinforced concrete structure.

6. The method according to any of the preceding claims, **characterized in that** step c) comprises applying a low-amplitude square wave at a specific frequency between the working electrode of the sensor and the frame (12).

7. The method according to claim 6, **characterized in that** the amplitude of the square wave is between 20 and 200 mV and the square wave is applied at a frequency between 1 and 10 KHz.

8. The method according to any of the preceding claims, **characterized in that** step d) comprises taking the potentiometric measurement at regular time intervals, and when the variation between the measured value and the earlier value is less than a threshold for 5 consecutive measurements, calculating the average of the 5 measurements and considering the average as the corrosion potential value.

9. The method according to any of the preceding claims, **characterized in that** the sensor is used as the working electrode, and the frame (12) as the reference electrode and auxiliary electrode.

10. The method according to any of claims 1 to 8, **characterized in that** the sensor is used as the working electrode, the frame (12) as an auxiliary electrode and a third electrode connected to the external connector as the reference electrode.

11. The method according to any of the preceding claims, **characterized in that** it further comprises step h) of sending the polarization resistance value and the intensity of corrosion value that are determined by the external measuring equipment to a central unit by means of a wireless connection.

**Patentansprüche**

1. Verfahren zum Bestimmen der Korrosion in einer bewehrten Betonstruktur unter Verwendung von mindestens einem Sensor, der in die bewehrte Betonstruktur eingebaut ist, wobei der Sensor umfasst:

- einen Träger (10), der den Sensor mit einer Stange des Rahmens (12) der Betonstruktur koppelt;
- ein Sensorelement (14), das mittels des Trägers (10) am Rahmen (12) befestigt ist;
- einen Temperatursensor (16);
- Leitungs- und Verbindungselemente (18), die das elektrische Isolieren oder Verbinden des Rahmens (12), des Sensorelements (14) und des Temperatursensors (16) mittels eines externen Verbindungsteils ermöglichen, das eingerichtet ist, um als elektrischer Schalter zu wirken;
- eine Metallverbindungsklemme (24), die den Rahmen (12) mit den Leitungs- und Verbindungselementen (18) verbindet;
- das externe Verbindungsteil, das mittels der Leitungs- und Verbindungselemente (18) am Sensorelement (14) und am Rahmen (12) angebracht ist und durch ein Verbindungsteil (20) im Inneren eines Anschlusskastens (22), der in die Oberfläche der Betonstruktur eingebettet ist, gebildet wird;

**dadurch gekennzeichnet, dass** das Verfahren die folgenden Schritte umfasst:

a) Verbinden eines externen Messgeräts mit dem externen Verbindungsteil des Sensors, Herstellen der Verbindung zwischen dem externen Messgerät und jedem der Sensorelemente (14), dem Temperatursensor (16) und dem Rahmen (12);
b) Bestimmen der Temperatur des Sensorelements (14) auf Basis der vom Temperatursensor (16) erhaltenen Messung;
c) Bestimmen des vom Beton übertragenen elektrischen Widerstands;

d) Bestimmen des Korrosionspotentialwerts mittels einer potentiometrischen Messung der Differenz des elektrischen Potentials zwischen einer Arbeitselektrode des Sensors und einer Referenzelektrode;

e) Anlegen von Impulsfolgen an den Sensor zur Berechnung des Polarisationswiderstands und der Korrosionsintensität;

f) Erhalten von Informationen von dem Sensor; und

g) Berechnen des Polarisationswiderstands und der Korrosionsintensität.

2. Verfahren nach Anspruch 1, **dadurch gekennzeichnet, dass** das Sensorelement (14) aus dem gleichen Material wie der Rahmen (12) der Betonstruktur hergestellt ist und eine Oberfläche von 5 bis 30 cm$^2$ aufweist.

3. Verfahren nach einem der vorhergehenden Ansprüche, **dadurch gekennzeichnet, dass** das externe Verbindungsteil ferner für die Verbindung der Referenzelektrode eingerichtet ist.

4. Verfahren nach einem der vorhergehenden Ansprüche, **dadurch gekennzeichnet, dass** der Temperatursensor (16) aus der Gruppe ausgewählt ist, bestehend aus einem Thermoelement, NTC, PTC, RTP oder einem Halbleitertemperatursensor.

5. Verfahren nach einem der vorhergehenden Ansprüche, **dadurch gekennzeichnet, dass** es unter Verwendung eines Sensornetzwerks durchgeführt wird, das aus einer Vielzahl von Sensoren gebildet ist, die an einer jeweiligen Vielzahl von Stellen im Rahmen (12) der bewehrten Betonstruktur angeordnet sind.

6. Verfahren nach einem der vorhergehenden Ansprüche, **dadurch gekennzeichnet, dass** Schritt c) das Anlegen einer Rechteckwelle mit niedriger Amplitude bei einer bestimmten Frequenz zwischen der Arbeitselektrode des Sensors und dem Rahmen (12) umfasst.

7. Verfahren nach Anspruch 6, **dadurch gekennzeichnet, dass** die Amplitude der Rechteckwelle zwischen 20 und 200 mV liegt und die Rechteckwelle mit einer Frequenz zwischen 1 und 10 kHz angelegt wird.

8. Verfahren nach einem der vorhergehenden Ansprüche, **dadurch gekennzeichnet, dass** Schritt d) das Durchführen der potentiometrischen Messung in regelmäßigen Zeitintervallen und, wenn die Abweichung zwischen dem gemessenen Wert und dem früheren Wert für 5 aufeinanderfolgende Messungen kleiner als ein Schwellenwert ist, das Berechnen des Durchschnitts der 5 Messungen und Betrachten des Durchschnitts als den Korrosionspotentialwert umfasst.

9. Verfahren nach einem der vorhergehenden Ansprüche, **dadurch gekennzeichnet, dass** der Sensor als Arbeitselektrode und der Rahmen (12) als Referenzelektrode und Hilfselektrode verwendet wird.

10. Verfahren nach einem der Ansprüche 1 bis 8, **dadurch gekennzeichnet, dass** der Sensor als die Arbeitselektrode, der Rahmen (12) als eine Hilfselektrode und eine dritte Elektrode, die mit dem externen Verbindungsteil verbunden ist, als die Referenzelektrode verwendet wird.

11. Verfahren nach einem der vorhergehenden Ansprüche, **dadurch gekennzeichnet, dass** es ferner Schritt h) des Sendens des Polarisationswiderstandswerts und des Korrosionsintensitätswerts, die von den externen Messgeräten bestimmt werden, an eine Zentraleinheit mittels einer drahtlosen Verbindung umfasst.

**Revendications**

1. Procédé de détermination de la corrosion dans une structure en béton armé au moyen de l'utilisation d'au moins un capteur incorporé dans la structure en béton armé, ledit capteur comprenant :

- un support (10) couplant le capteur à une barre du cadre (12) de la structure en béton ;
- un élément capteur (14) fixé au cadre (12) au moyen du support (10) ;
- un capteur de température (16) ;
- des éléments de conduction et de connexion (18) qui permettent d'isoler ou de connecter électriquement le cadre (12), l'élément capteur (14) et le capteur de température (16) au moyen d'un connecteur externe agencé pour agir comme un interrupteur électrique ;
- une pince de connexion métallique (24) reliant le cadre (12) aux éléments de conduction et de connexion (18) ;

- le connecteur externe fixé à l'élément capteur (14) et au cadre (12) au moyen des éléments de conduction et de connexion (18) et formé par un connecteur (20) à l'intérieur d'une boîte de connexion (22) noyée dans la surface de la structure en béton ;

**caractérisé en ce que** le procédé comprend les étapes de :

a) connexion d'un équipement de mesure externe au connecteur externe du capteur, établissement de la connexion entre l'équipement de mesure externe et chacun de l'élément capteur (14), du capteur de température (16) et du cadre (12) ;
b) détermination de la température de l'élément capteur (14) sur la base de la mesure obtenue à partir du capteur de température (16) ;
c) détermination de la résistance électrique conférée par le béton ;
d) détermination de la valeur du potentiel de corrosion au moyen d'une mesure potentiométrique de la différence de potentiel électrique entre une électrode de travail du capteur et une électrode de référence ;
e) application de trains d'impulsions au capteur, pour calculer la résistance de polarisation et l'intensité de la corrosion ;
f) obtention d'informations du capteur ; et
g) calcul de la résistance de polarisation et de l'intensité de la corrosion.

2. Procédé selon la revendication 1, **caractérisé en ce que** l'élément capteur (14) est fabriqué dans le même matériau que le cadre (12) de la structure en béton et a une surface mesurant de 5 à 30 cm$^2$.

3. Procédé selon l'une quelconque des revendications précédentes, **caractérisé en ce que** le connecteur externe est en outre agencé pour la connexion de l'électrode de référence.

4. Procédé selon l'une quelconque des revendications précédentes, **caractérisé en ce que** le capteur de température (16) est choisi dans le groupe constitué par un thermocouple, NTC, PTC, RTP ou un capteur de température à semi-conducteur.

5. Procédé selon l'une quelconque des revendications précédentes, **caractérisé en ce qu'**il est réalisé au moyen de l'utilisation d'un réseau de capteurs formé par une pluralité de capteurs agencés en une pluralité d'emplacements respectifs dans le cadre (12) de la structure en béton armé.

6. Procédé selon l'une quelconque des revendications précédentes, **caractérisé en ce que** l'étape c) comprend l'application d'une onde carrée de faible amplitude à une fréquence spécifique entre l'électrode de travail du capteur et le cadre (12).

7. Procédé selon la revendication 6, **caractérisé en ce que** l'amplitude de l'onde carrée est comprise entre 20 et 200 mV et l'onde carrée est appliquée à une fréquence comprise entre 1 et 10 KHz.

8. Procédé selon l'une quelconque des revendications précédentes, **caractérisé en ce que** l'étape d) comprend la prise de la mesure potentiométrique à intervalles de temps réguliers et, lorsque l'écart entre la valeur mesurée et la valeur antérieure est inférieur à un seuil pour 5 mesures consécutives, le calcul de la moyenne des 5 mesures et la considération de la moyenne comme valeur de potentiel de corrosion.

9. Procédé selon l'une quelconque des revendications précédentes, **caractérisé en ce que** le capteur est utilisé comme électrode de travail et le cadre (12) comme électrode de référence et électrode auxiliaire.

10. Procédé selon l'une quelconque des revendications 1 à 8, **caractérisé en ce que** le capteur est utilisé comme électrode de travail, le cadre (12) comme électrode auxiliaire et une troisième électrode connectée au connecteur externe comme électrode de référence.

11. Procédé selon l'une quelconque des revendications précédentes, **caractérisé en ce qu'**il comprend en outre l'étape h) d'envoi de la valeur de résistance de polarisation et de l'intensité de la valeur de corrosion qui sont déterminées par l'équipement de mesure externe à une unité centrale au moyen d'une connexion sans fil.

**FIG. 1**

**FIG. 2**

**FIG. 3**

**FIG. 4**

FIG. 5

FIG. 6

**FIG. 7**

Reinforced concrete element

Connection box

Sensor

Structure

Reference / Aux

External reference

Contact of the reference

Ecorr (V)

ΔE (V)

CORRECTED
Ecorr (V)

0.00 m

Buried region

Sensor element

- X m

**FIG. 8**

**FIG. 9**

**FIG. 10**

EP 3 293 509 B1

FIG. 11

FIG. 12

FIG. 13

FIG. 14

**FIG. 15**

**FIG. 16**

FIG. 17

FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 103852488
- JP 2007240481 B
- JP 2011089871 B
- JP 2014062782 B

**Non-patent literature cited in the description**

- A quantitative study of concrete-embedded steel corrosion using potentiostatic pulses. **BASTIDAS et al.** Corrosion. National Assoc. of Corrosion Engineers International, December 2007, vol. 63, 1094-1100